# EUROPÄISCHE PATENTANMELDUNG

(11) **EP 1 288 837 A2**
(43) Veröffentlichungstag der Anmeldung: **05.03.2003**
(21) Anmeldenummer: 02018378.6
(22) Anmeldetag: 14.08.2002
(51) Int. Cl.: G06F 19/00

(54) **Qualitätskontrolle in disease management services**

(30) Priorität: 27.08.2001 DE 10141832
(71) Anmelder: SIEMENS AKTIENGESELLSCHAFT, 80333 München (DE)
(72) Erfinder: Abraham-Fuchs, Klaus, 91058 Erlangen (DE); Bieger, Johannes, Dr., 80638 München (DE); Rumpel, Eva, Dr., 91052 Erlangen (DE); Schmidt, Kai-Uwe, Dr., Paoli, PA 19301 (US); Tietze, Daniel, 91080 Spardorf (DE)

(57) **Zusammenfassung**

Qualitätskontrollsystem in Disease Management Services zur Aufklärung, Weiterbildung und Motivierung von Patienten in Verbindung mit Telemonitoring kritischer Körperwerte und daraus resultierender frühzeitiger Erkennung von Risikosituationen, wobei über Zufallsgeneratoren aus den zulässigen Patientendaten ein virtueller Patient erstellt wird, wobei aus den vorhandenen Hardwarekomponenten eine zufällige Auswahl getroffen wird und wobei ebenfalls zufallsgeneriert aus einer Liste möglicher Ereignisse im Disease Management Prozess ein zufälliges Ereignis ausgewählt wird, wobei zum Aufspüren von Fehlern die Systemreaktion beim Durchspielen dieses Ereignisses von einem Expertensystem anhand der ihm bekannten Sollantworten bewertet wird.

## Beschreibung

Die Erfindung bezieht sich auf ein Qualitätskontrollsystem in Disease Management Services zur Aufklärung, Weiterbildung und Motivierung von Patienten in Verbindung mit Telemonitoring kritischer Körperwerte und daraus resultierender frühzeitiger Erkennung von Risikosituationen.

Disease Management Services Provider betreuen typischer Weise Patienten, die an einer chronischen Volkskrankheit leiden, wie zum Beispiel Diabetes, Asthma oder Hypertonie. Damit werden große Patientenzahlen mit einem weitgehend standardisierten Behandlungsplan über lange Zeiträume, typischer Weise Monate oder Jahre, betreut, was zu einer deutlich gesteigerten Kosteneffizienz gegenüber traditioneller Patientenversorgung führt.

Eine wirksame Kosteneffizienz wird unter anderem durch eine möglichst weitgehende Automatisierung der Patientenbetreuung erreicht. So werden zum Beispiel in festgelegten Intervallen automatisch Patienteninformationsmaterial verschickt oder Beratungsanrufe durchgeführt, oder es werden Messwerte, zum Beispiel Blutdruck vom Patienten in digitaler Form an das Kontrollzentrum gesendet, dort automatisch bewertet und bei Über- oder Unterschreitung von Grenzwerten automatisch Benachrichtigungen mit einer entsprechenden Behandlungsempfehlung per Fax oder Email an den betroffenen Arzt oder den Patienten versendet.

Wie bei allen hochautomatisierten Prozessen ist auch hier eine systematische Qualitätskontrolle dieses telemedizinischen Disease-Management-Prozesses notwendig, um Fehler zu vermeiden bzw. rechtzeitig aufzudecken. Mögliche Fehlerquellen reichen von defekten Kabeln oder Rechnern über falsch eingegebene Rufnummern bis zur Verfälschung oder Verlust bei digitaler Datenübertragung.

Aus der EP 0 917 078 A1 ist ein Disease-Management-Sytem bekannt, wobei jedoch dort keine Kontrolle der Funktion dieses Systems vorgesehen ist.

Die DE 196 51 334 A1 beschreibt ein Betriebstestverfahren und eine hierfür geeignete Vorrichtung, um einen Betriebstest für ein testendes System durchzuführen. Dort wird aber gerade nicht das reale System selbst, sondern nur ein Testsystem durch gespielt, sodass die in der Praxis auftretenden Fehler im Disease-Management-Servicesystem mit einem solchen Testsystem gerade nicht erkannt werden können.

Der Erfindung liegt deshalb die Aufgabe zugrunde, ein Qualitätskontrollsystem zu schaffen, das es gestattet, automatisch Fehler in den unterschiedlichsten Bereichen und Stufen eines solchen Disease Management Services aufzudecken und zu korrigieren.

Zur Lösung dieser Aufgabe ist erfindungsgemäß vorgesehen, dass über Zufallsgeneratoren aus den zulässigen Patientendaten ein virtueller Patient erstellt wird, dass aus den vorhandenen Hardwarekomponenten eine zufällige Auswahl getroffen wird und dass ebenfalls zufallsgeneriert aus einer Liste möglicher Ereignisse im Disease Management Prozess ein zufälliges Ereignis ausgewählt wird, wobei zum Aufspüren von Fehlern die Systemreaktion beim Durchspielen dieses Ereignisses von einem Expertensystem anhand der ihm bekannten Sollantworten bewertet wird.

Das erfindungsgemäße Qualitätskontrollsystem ähnelt der Qualitätsprüfung von Software mit komplexen Benutzer-Interfaces. Dort wird teilweise Testsoftware eingesetzt, die auf dem Prinzip der statistischen Simulation möglicher realer Bedienungsabläufe beruht: In verzweigten Eingabebäumen wird willkürlich und zufallsverteilt mit einem beliebigen Eingabewert gestartet. Diese Eingabe führt auf dem nächsten Verzweigungslevel zu neuen Optionen möglicher Eingaben, für die wieder ein (zulässiger) Eingabewert zufallsbedingt gewählt wird. So kann das System in beliebigen Szenarios, insbesondere auch in unter realen Bedingungen seltenen Szenarios, durchgespielt werden und auf Stabilität und Fehlermeldungen geprüft werden. Dieses Testkonzept wird erfindungsgemäß auf Disease Management Services übertragen. Da im Gegensatz zu einmal fertiggestellter und von Fehlern befreiter Software Fehler beim Disease Management Services ständig neu auftreten können, muss hier eine Funktionsprüfung kontinuierlich über den gesamten Betriebszeitraum hinweg erfolgen, weshalb derartige Testläufe mit virtuellen Patienten in vorgegebenen Zeitabständen automatisch durchgeführt werden sollen.

Die zulässigen Patientendaten, beispielsweise Geburtsdatum, Grunderkrankung, von Arzt verordnete Messwerte für Monitoring, betreuender Hausarzt usw., die zulässigen Hardwarekomponenten - eigener Patientenrechner, Faxgeräte, Telefonverbindungen, Server usw. sowie eine Liste möglicher Ereignisse im Disease Management Prozess, zum Beispiel Fälligkeitsdatum für eine bestimmte Informationsschrift, Anruf eines Patienten im Call-Center über bestimmte Art von Beschwerden, elektronisch übersandter Blutdruckwert usw. sollen dabei in Weiterbildung der Erfindung in getrennten Datenbanken gespeichert sein.

Um dabei verstärkt auch reale früher aufgetretener Fehler mit berücksichtigen zu können und nicht nur zufallsgeneriert aus Listen aller nur denkbaren Ereignisse und Personendaten oder Hardwarekomponenten einen Zufallstestlauf zu erzeugen, kann in Ausgestaltung der Erfindung auch vorgesehen sein, dass neben der zufallsgenerierten Auswahl von Ereignissen reale Fälle aus der Vergangenheit und/oder reale Fehlerfälle aus der Vergangenheit und/oder reale oder fingierte, durch Fachexperten zusammengestellte Fälle regelmäßig oder zufallsgeneriert durchgespielt werden.

Die Mitteilungen des Systems sollen dabei je nach ihrer Bedeutung als Testanfragen gekennzeichnet sein, damit nicht lediglich aufgrund eines durchgespielten Tests mit einem virtuellen Patienten ein Notarzt zu diesem Patienten geschickt wird. Der Test soll ja lediglich ergeben, ob die Anfrage und die in die Anfrage verwickelten Hardwarekomponenten, also die technischen Übermittlungsgeräte usw., tatsächlich funktionieren und ob - was für die Funktion des erfindungsgemäßen Qualitätskontrollsystem ebenfalls von Bedeutung ist, auch mögliche menschliche Fehlerquellen ausgeschlossen werden müssen. Zu diesem Zweck soll neben der Überprüfung technischer Funktionen, insbesondere also der bereits angesprochenen Hardwarekomponenten, auch eine Überprüfung und Bewertung möglicher menschlicher Fehlerquellen, wie Reaktionszeit auf Mitteilungen oder dergleichen, erfolgen. Erfolgt nämlich keine oder keine rechtzeitige Reaktion des Patienten, eines Arztes oder eines anderen Systemgliedes, so bedarf es gegebenenfalls der Überprüfung, ob der betreffende Arzt weiter in das Disease Management Service System einbezogen werden kann oder gegebenenfalls wegen Unzuverlässigkeit ausgeschlossen und durch einen anderen Arzt ersetzt werden muss.

Die Testläufe und -ergebnisse sollen für eine Bewertung von Geschäftssegmenten oder dergleichen gespeichert werden, wobei in jedem Fall das Bewertungssystem mit einem Meldesystem versehen sein sollte, das im Bedarfsfall Techniker, Ärzte oder einen Qualitätsbeauftragten informiert oder befragt.

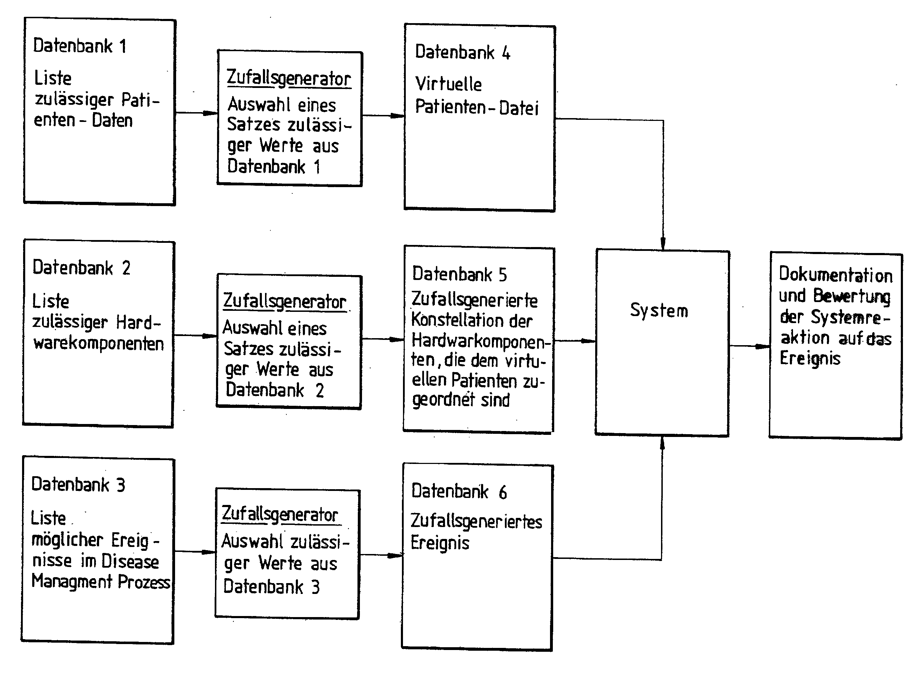

Weitere Vorteile, Merkmale und Einzelheiten der Erfindung ergeben sich aus der nachfolgenden Beschreibung einiger Ausführungsbeispiele sowie anhand der Zeichnung, die ein Ablaufdiagramm des erfindungsgemäßen Qualitätskontrollsystems zeigt.

In drei Datenbanken sind Listen einmal zulässiger Patientendaten, einmal zulässiger Hardwarekomponenten und zum Dritten mögliche Ereignisse im Disease Management Prozess gespeichert, aus denen jeweils ein Zufallsgenerator einen Satz zulässiger Werte auswählt und weiteren Datenbanken 4 bis 6 zuführt. Dem virtuellen Patienten wird über eine zufallsgenerierte Konstellation der Hardwarekomponenten das vom Zufallsgenerator aus der Datenbank 3 herausgegriffene zufallsgenerierte mögliche Ergebnis im Disease Management Prozess zugeordnet und die Systemreaktion beim Durchspielen dieses Ereignisses dokumentiert und bewertet. Im Einzelnen kann dies z. B. wie nachstehend beschrieben. erfolgen:

Diese Anforderungen erfüllt die Erfindung auf folgende Art und Weise:
- In der Patientenkartei des Disease Management Service Providers wird ein "virtueller Patient" angelegt. Alle "statistischen" (d. h. für diesen Patienten charakteristischen und unveränderlichen) Informationseinheiten (z. B. Geburtsdatum, Grunderkrankung, vom Arzt verordnete Messwerte für Monitoring, betreuender Hausarzt) dieser Patientendatei werden von einem Zufallsgenerator aus einer Liste zulässiger Werte ausgewählt.
- eine weitere Datei ordnet dieser "virtuellen Patientendatei" ein Netzwerk von Hardwarekomponente zu, die für die Betreuung dieses Patienten notwendig sind (z. B. Fax Nr. 25, Call-Center Hamburg Nord, Server Nr. 32, Back-Up-Rechner Nr. 8), wieder per Zufallsgenerator ausgewählt aus einer Liste real existierender und zulässiger Hardware-Komponenten.
- eine dritte Datei enthält Informationselemente über mögliche Ereignisse im Disease Management Prozess (Fälligkeitsdatum für Informationsschrift Nr. 3; Anruf des Patienten im Call-Center: "Habe akute Atembeschwerden"; elektronischer übersandter Blutdruckwert etc.). Auch die Ereignisart und dem Ereignis zugeordnete Werte (Blutdruck 90/180) werden per Zufallsgenerator gewählt.

Eine solcherart zufällige Konstellation aus Patientendaten, Hardware-Konfiguration und Ereignis kann beliebig häufig (stündlich, täglich, wöchentlich) neu generiert werden.

Das Auftreten des zufällig generierten "Ereignisses" löst nun (vor dem Hintergrund ebenfalls zufällig generierter Patientengrunddaten und innerhalb des Netzwerkes der zufällig gewählten Hardware-Konfiguration) eine Systemreaktion aus.

### Beispiele:

- der zufallsgenerierte Blutdruckwert überschreitet einen zugelassenen Grenzwert und löst automatisch eine Email oder einen Fax-Hinweis an den betreuenden Arzt aus, wobei dieser Hinweis bevorzugt als Testanfrage gekennzeichnet ist.
- das angeforderte Informationsdokument wird dem "virtuellen Patienten" zugestellt.

In gewissem Umfang kann auch die ausgelöste Systemreaktion nur simuliert werden, indem statt der Postauslieferung eines Briefumschlages eine Email versandt wird, deren Eingang am Testrechner automatisch dokumentiert wird, und somit der gesamte Qualitäts-Prüfungs-Prozess vollständig digital simuliert, ausgewertet und dokumentiert werden etwa dergestalt: Das zufallsgenerierte Ereignis XYZ hat unter den Randbedingungen a, b und c die erwartete Systemreaktion ACD im Zeitraum x Stunden ausgelöst.

Andere Simulationsszenarien prüfen zusätzlich zu rein technischen Funktionen auch mögliche menschliche Fehlerquellen (reagiert der Vertragsarzt auf das Hinweisfax in angemessenem Zeitrahmen mit der erwarteten Aktion). Gegebenenfalls können solche Aktionen eines involvierten Menschen im Prozess nicht mehr komplett automatisch erfasst und dokumentiert werden, sondern müssen von einem Qualitätsbeauftragten entgegengenommen, bewertet und dokumentiert werden. Das vorgeschlagene System ist somit in der Lage, in beliebigen Zeitintervallen nahezu beliebig viele Prozesskonstellationen innerhalb des gesamten Disease Management Services (involvierte Software, Geräte und Menschen) zu prüfen und zu dokumentieren. Insbesondere kann hiermit auch die Prozessreaktion auf in der Realität besonders seltenen Notfallsituationen, wie das Auftreten eines Herzinfarkts, Delirium bei extrem hohen Blutzuckerwerten usw., geprüft werden.

Daraus kann eine Qualitätsstatistik abgeleitet werden, die Ausfallraten, Fehlreaktionen usw. mit einer statistisch relevanten Aussagekraft quantifizieren und darstellen kann. So kann mithilfe einer solchen Statistik zum Beispiel auch die postitive Wirkung einer Änderung im Prozess (zum Beispiel Ersetzen von fehlerhaften Faxgeräten durch eine neue Produktgeneration in der Niederlassung X, Wechsel des Email Providers usw.) oder Vergleiche von Geschäftssegmenten (Betreuung der Patienten im Großraum der Städte X und Y) quantifiziert und dokumentiert werden.

Zum noch besseren Verständnis der Erfindung wird nachstehend ein konkretes Ausführungsbeispiel einer Zufallskonstellation eines virtuellen Patienten beschrieben:

Ein Patient mit dem virtuellen Namen Peter Müller leidet an Diabetes Typ II. Dem Patienten ist ein digital auslesbares Blutzuckermessgerät zugeordnet, mit der Vorgabe, mindestens 5 mal täglich den Blutzucker zu messen. Die gemessenen Werte werden per e-mail mit der virtuellen Absender-Adresse peter.müller@t-online.de an einen zentralen Server gesandt und dort automatisch mit einer oberen und unteren Schwellwert-Regel bewertet. Bei Über- oder Unterschreitung des Schwellwertes wird automatisch ein FAX an den innerhalb des Disease-Management-Services für Herrn Müller zuständigen Hausarzt, Herrn Meier, versendet, mit der Aufforderung, Herrn Müller unverzüglich zu kontaktieren und sicherzustellen, dass notwendige medizinische Maßnahmen eingeleitet werden (Z. B. Insulinspritze bei Überzuckerung). Die Vorgabe für den Hausarzt ist, zuerst Herrn Müller telefonisch zu erreichen, um zu prüfen, ob Herr Müller die Situation selbst in den Griff bekommen kann. Kann er Herrn Müller nicht erreichen, so muss unverzüglich ein Notarzt zu Herrn Müller geschickt werden, da er davon ausgehen muss, dass Herr Müller einen hyperglykämischen Schock erlitten hat.

In dem virtuellen konstruierten Fallbeispiel wird der Blutzuckerwert vom Qualitätssicherungsprogramm zufällig generiert, im vorliegenden Fall mit 220 mg/dl (Hyperglykämie). Der Wert wird vom virtuellen e-mail Absender peter.müller@t-online.de dem zentralen Server zugespielt.

Die bei diesem virtuellen Patienten beteiligten SystemKomponenten, die in diesem Fall im Zusammenspiel überprüft werden, sind demnach:
- e-mail-Eingang des zentralen Servers
- Auswertesoftware des zentralen Servers
- FAX-Sender des zentralen Servers an Arztpraxis Meier
- Reaktion des Hausarztes Herrn Meier
- Telefonverbindung Arztpraxis zum Testrechner

Trifft der Anruf des Hausarztes binnen einer vorgegebenen Zeit, z. B. 5 Minuten, beim Testrechner ein (von dem der Hausarzt mittels einer automatischen Ansage erfährt, das es sich um einen Testfall zur Qualitätssicherung handelt), so dokumentiert das System den Testfall und die ordnungsgemäße Funktion alle beteiligen Komponenten. Trifft der Anruf nicht ein, so wird der virtuelle Fehlerfall an das Servicepersonal weitergeleitet, welches nun mittels der Systemprotokolle etc. das fehlerhafte Glied der Systemkette aufspüren und den Fehler beheben muss.

## Patentansprüche

1. Qualitätskontrollsystem in Disease Management Services zur Aufklärung, Weiterbildung und Motivierung von Patienten in Verbindung mit Telemonitoring kritischer Körperwerte und daraus resultierender frühzeitiger Erkennung von Risikosituationen, **dadurch gekennzeichnet, dass** über Zufallsgeneratoren aus den zulässigen Patientendaten ein virtueller Patient erstellt wird, dass aus den vorhandenen Hardwarekomponenten eine zufällige Auswahl getroffen wird und dass ebenfalls zufallsgeneriert aus einer Liste möglicher Ereignisse im Disease Management Prozess ein zufälliges Ereignis ausgewählt wird, wobei zum Aufspüren von Fehlern die Systemreaktion beim Durchspielen dieses Ereignisses von einem Expertensystem anhand der ihm bekannten Sollantworten bewertet wird.

2. Qualitätskontrollsystem nach Anspruch 1, **dadurch gekennzeichnet, dass** die zulässigen Patientendaten, die zulässigen Hardware-Komponenten und eine Liste möglicher Ereignisse im Desease Management Prozess in getrennten Datenbanken gespeichert sind.

3. Qualitätskontrollsystem nach Anspruch 1 oder 2, **dadurch gekennzeichnet, dass** neben der zufallsgenerierten Auswahl von Ereignissen reale Fälle aus der Vergangenheit und/oder reale Fehlerfälle aus der Vergangenheit und/oder reale oder fingierte durch Fachexperten zusammengestellte Fälle regelmäßig oder zufallsgeneriert durchgespielt werden.

4. Qualitätskontrollsystem nach einem der Ansprüche 1 bis 3, **dadurch gekennzeichnet, dass** die Testläufe mit virtuellen Patienten in vorgegebenen Zeitabständen automatisch durchgeführt werden.

5. Qualitätskontrollsystem nach einem der Ansprüche 1 bis 4, **dadurch gekennzeichnet, dass** die Mitteilungen des Systems als Testanfragen gekennzeichnet sind.

6. Qualitätskontrollsystem nach einem der Ansprüche 1 bis 5, **dadurch gekennzeichnet, dass** neben der Überprüfung technische Funktionen, insbesondere der Hardware-Komponenten- auch eine Überprüfung und Bewertung möglicher menschlicher Fehlerquellen, wie Reaktionszeit auf Mitteilungen oder dergleichen, erfolgt.

7. Qualitätskontrollsystem nach einem der Ansprüche 1 bis 6, **dadurch gekennzeichnet, dass** die Testläufe und -ergebnisse für eine Bewertung von Geschäftssegmenten oder dergleichen gespeichert werden.

8. Qualitätskontrollsystem nach einem der Ansprüche 1 bis 7, **dadurch gekennzeichnet, dass** das Bewertungssystem mit einem Meldesystem versehen ist, das im Bedarfsfall Techniker, Ärzte oder einen Qualitätsbeauftragten informiert oder befragt.
